# EUROPEAN PATENT APPLICATION

(11) **EP 1 223 175 A1**
(43) Date of publication of application: **17.07.2002**
(21) Application number: 00961240.9
(22) Date of filing: 26.09.2000
(51) Int. Cl.: C07K 5/075, A23L 1/236, A23L 1/22, A23L 2/60

(54) **SOLID SWEETENER COMPOSITIONS, LIQUID SWEETENER COMPOSITIONS AND UTILIZATION THEREOF**

(30) Priority: 05.10.1999 JP 28434599; 05.10.1999 JP 28434499
(71) Applicant: Ajinomoto Co., Inc., Tokyo 104-8315 (JP)
(72) Inventor: ISHII, Shoichi, Food R&D Lab., Ajinomoto Co., Inc., Kawasaki-shi, Kanagawa-ken 210-0801 (JP)
(74) Representative: Nash, David Allan
(86) International application number: PCT/JP00/06629
(87) International publication number: WO 01/25263

(57) **Abstract**

Solid compositions of sweetener with a high intense sweetness (including those in the form of solid food) having a sweetness quality with homogeneous dispersion in the whole, which comprise an aspartyl dipeptide ester derivatives such as N-[N-[3-(3-hydroxy-4-methoxyphenyl) propyl] -L-α-aspartyl]-L-phenylalanine 1-methyl ester which is a sweetener with a high intense sweetness (one kind or more; which may be in the salt form), and a filler (including a bulking agent, a carrier and so on), particularly at least one of the compounds contained in the group consisting of sugar , sugar alcohol, oligosaccharide and polysaccharide, and are produced by a process involving the step of mixing at least said derivative(s) in the form of solution during manufacture, are provided.

Solutions or suspensions with a high intense sweetness, stably over a long period of time, which are obtained by mixing at least one kind of said aspartyl dipeptide ester derivative having a high intense sweetness in an edible medium, preferably together with at least one of the compounds contained in the group consisting of sugar, sugar alcohol and oligosaccharide as a stabilizer, and homogeneously dissolving or suspending, are provided. These solutions and so on are useful as sweeteners, and for imparting sweetness to food and drink which are added sweetness or the product in need of sweetness. Liquid compositions of sweetener with a high intense sweetness, which are stable over a long period of time, excellent in solubility and workability without undergoing scattering, can be obtained.

## Description

### Technical Field

The present invention relates to a novel solid sweetener composition, more specifically, a solid sweetener composition (including a solid food and so on having a sweetness) which comprises the specific novel aspartyl dipeptide ester derivative which is a sweetener with a high intense sweetness, for example, N-[N-[3-(3-hydroxy-4-methoxyphenyl) propyl]-L-α-aspartyl]-L-phenylalanine 1-methyl ester (referred to as "derivative 1") and a filler, thereby said derivative being dispersed homogeneously, and food and drink and so on using the same.

In addition, the present invention relates to a novel liquid sweetener composition, more specifically, a liquid sweetener composition comprising a solution which dissolves at least one of said specific novel aspartyl dipeptide ester derivative, for example, derivative 1 and so on in the edible medium, preferably in the form of solution or suspension dispersing or dissolving said derivative homogeneously and stably, and more preferably a liquid composition of sweetener with a high intense sweetness (including those in the form of food and drink such as a sweetener) comprising at least one of the compounds contained in the group consisting of sugar, sugar alcohol, and oligosaccharide as a stabilizer therefor, and food and drink using the same, and the another sweetened product and so on.

### Background Art

It has been reported that the sweetness intensity of Neotame which is a sweetner with a high intense sweetness is 10000 times that of sucrose by weight (refer to Japanese Patent Kohyo Publication JP-A-8-503206), and the sweetness intensity of Aspartame is 200 times that of sucrose by weight (refer to Japanese Patent Kokoku Publication JP-B-47-31031). These sweeteners have been commercially used already, or their research for a practical application is going on. Besides, although many other sweeteners with a high intense sweetness having a sweetness quality with a high intense sweetness have been proposed, there are many problems for practical use.

It is still desired to develop a sweetener with a high intense sweetness which is high in the intense sweetness and different from the conventional sweeteners, and a sweetener substance with a high intense sweetness which has a preferable sweetness quality and an excellent physical property such as stability and so on, or a highly practical composition of sweetener with a high intense sweetness which comprises such sweetener substance with a high intense sweetness.

### Problem to be solved by the Invention

As a result of extensive research to develop a sweetener with a high intense sweetness, which has a high intense sweetness, it was found that the aspartyl dipeptide ester derivative represented by the following general formula (2) had a high intense sweetness and said derivative was useful as a sweetener with a high intense sweetness. The patent application on these contents (inventions) has been already filed by the present applicant.

According to a finding of the present inventor, the magnification of sweetness intensity of said derivative is extremely high, and it is found that, when the derivative as such is directly used for food and so on in need of sweetness, for example, by sprinkling, the sweetness thereof is not mixed therewith homogeneously due to the extremely high magnification of sweetness intensity or so, to give the food and so on having unhomogeneous sweetness, which is not preferable. Then, when the sweetener composition was prepared by mixing with a filler, it was confirmed that the mixing of the two substances in the ordinary powder form each together did not make the derivative to a homogeneous dispersion or mixture, and therefore the use of this derivative as it is was not preferable due to the unbalanced sweetness.

Therefore, when a solid sweetener composition is produced, the method for dispersing and mixing the sweetness homogeneously is necessary. Thus, it is an object of the present invention to develop a solid sweetener composition in which said derivative can be homogeneously dispersed and mixed.

Next, as a result of further studies by the present inventor on said aspartyl dipeptide ester derivative, the magnification of sweetness intensity of said derivative was confirmed to be extremely high and 5000 to 50000 times (such times or more) that of sucrose, and the use of the same as a sweetener with a high intense sweetness for various kinds of food and drink has been tried.

It was found that said derivative was satisfied sufficiently in the aspects of intense sweetness, sweetness quality and diet and so on, however, it caused some troubles due to the intrinsic physical property in the aspartyl dipeptide ester derivative, when the same in the powder of raw material was used as it was, and therefore the derivative was difficult to handle. For example, it has a characteristics of powder such as a large specific volume and being easy to spread out, because most of its crystals are fine and needle-like. Thus, it is in danger to make worse the working environment by spreading out the derivative during handling operation. At the same time, it is easy to lose the derivative by spreading out.

On using the derivative for various kinds of food and drink, the present inventor has found that the aqueous solution with a high intense sweetness was stable and useful for a liquid sweetener composition. As a result of further studies thereby, it was revealed as an object (problem) that an improvement of the solubility, the dispersibility and the stability in the edible medium by, for example, improving the solubility in the water itself, to make simple the operation of solubilization in water by preventing the formation of coagulation or so on (the state where the powder particles are turned into a solid mass) or to prevent it from the formation of formimg phenomena and so on, made it extremely profitable in the food processing.

On the other hand, as an intention to health, diet and so on becomes higher, it has been in demand to develop a non-sugar sweetener in the low-calorie which substitutes for sucrose. In addition, it is also an important object to develop means to solve the conventional problems in the sucrose containing products such as browning, stickiness and so on, or a sweetener suitable for the production of drink, sherbet (ice block) and so on. In the production of drink, sherbet and so on, not only the influence of the sweetener on the quality of end product, but also the operational efficiency in the production process and changes in physical property and so on resulted from the sweetener are the important requisites (factors) to select a sweetener.

Further, when the condensed stock solution for drink and so on was previously prepared, and the dilution or bottling of the stock solution was separately performed, or it was supplied as the stock solution or the syrup for vending machine, it is preferable to reduce the volume as little as possible for the sake of convenience of transport, storage and so on, and it is desirable to develop such a composition of a sweetener with a high intense sweetness.

Under the situations as described above, a composition of sweetener with a high intense sweetness comprising said aspartyl dipeptide ester derivative stably, which can be handled easily without spreading out, has been in demand.

Thus, it is also an object of the present invention to provide and obtain a sweetener composition which is sufficient in view of not only the quality of end product, but also process control and operational efficiency during the production and delivery in terms of said derivative.

For that purpose, the method to solve the following concrete problems must be developed.
1. Prevention of the spreading out of said aspartyl dipeptide ester derivative.
2. Improvement of solubility for easy to dissolution.
3. A composition of sweetener with a high intense sweetness which can be stored in a small place, can impart a homogeneous sweetness and be stable for a long time (high solubility and high dispersibility).

### Disclosure of the Invention

The present inventor has studied eagerly to solve the problems described above and has found that a solid sweetener composition comprising at least said derivative with a high intense sweetness and a solid filler can be obtained by the process comprising at least a step of mixing said derivative in the form of a solution during manufacture, thereby said derivative being mixed and dispersed homogeneously. This finding has led to the accomplishment of one of the invention "a solid sweetener composition" included in the present invention as one embodiment thereof.

That is, the present invention lies in a solid sweetener composition comprising an aspartyl dipeptide ester derivative (which may be in the salt form) represented by the following general formula (2), more preferably the following general formula (1), and a solid filler, wherein the process of producing said composition comprises at least a step of mixing said derivative in the form of a solution during manufacture, thereby said derivative being mixed and dispersed homogeneously.

Said aspartyl dipeptide ester derivative used for the solid sweetener composition in the present invention includes those in the form of salts, and only one derivative or a mixture of not less than two derivatives is used. 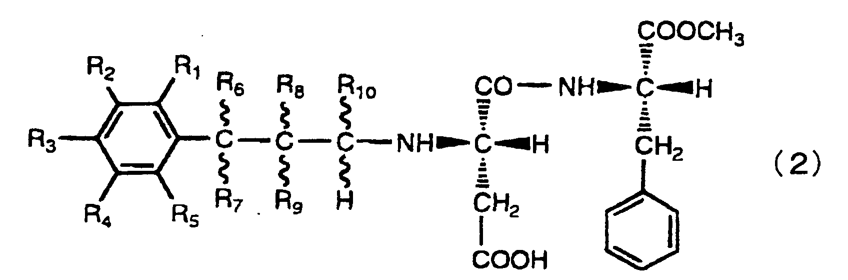 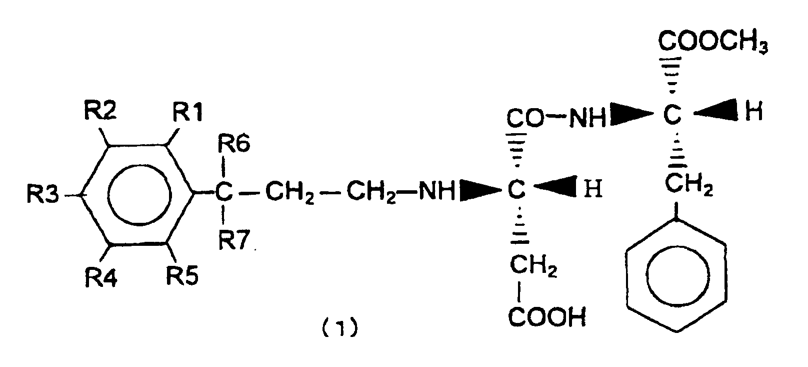 wherein in the above formulae R₁, R₂, R₃, R₄ and R₅ are independent from each other, and each denotes any one selected from the group consisting of a hydrogen atom, a hydroxyl group, an alkoxy group having 1 to 3 carbon atoms (methoxy, ethoxy, n-propoxy and so on) , an alkyl group having 1 to 3 carbon atoms (methyl, ethyl, n-propyl and so on) and a hydroxyalkyloxy group having 2 or 3 carbon atoms (O(CH₂)₂OH, OCH₂CH(OH)CH₃ and so on), and herein as for R₁ and R₂, or R₂ and R₃, the respective two symbols (R₁ and R₂, or R₂ and R₃), combined together with each other may form a methylene dioxy group (OCH₂O).

R₆, R₇, R₈, R₉ and R₁₀ are independent from each other, and each denotes a hydrogen atom or an alkyl group having 1 to 3 carbon atoms (a methyl, an ethyl, an isopropyl group and so on), respectively, herein any two substituents optionally selected from the group consisting of R₆, R₇, R₈, R₉ and R₁₀, combined together with each other, may form an alkylene group having 1 to 5 carbon atoms (CH₂, CH₂CH₂, CH₂CH₂CH₂ and so on).

When R₆ and R₇, or R₈ and R₉ denote different substituents with each other, or R₁₀ denotes an substituent except for a hydrogen atom, the configuration of the carbon atom to which these substituents (R₆ and R₇, R₈ and R₉ or R₁₀) are linked, has no restriction, and may be any one of (R), (S) and (RS) or mixture thereof, for example. In addition, wiggly lines described as the bond of R₆ to R₁₀, and a hydrogen atom with a carbon atom in said general formula (2), means that the direction of the bond is free (is not specified).

However, the derivative in which R₆ denotes a hydrogen atom or a methyl group and R₁, R₂, R₃, R₄, R₅, R₇, R₈, R₉ and R₁₀ denote a hydrogen atom concurrently is excluded. The derivative in which R₂ or R₄ denotes a methoxy group, R₃ denotes a hydroxyl group, R₁₀ denotes a hydrogen atom or a methyl group, and R₁, R₄, R₅, R₆, R₇, R₈, and R₉ denote a hydrogen atom concurrently is also excluded.

As an aspartyl dipeptide ester derivative used for the solid sweetener composition of the present invention, the derivative represented by said formula wherein all of R₈, R₉ and R₁₀ denote a hydrogen atom is more preferable.

The sweetness intensity of the aspartyl dipeptide ester derivative used for the solid sweetener composition of the present invention is preferably more than 4,000 times that of sucrose.

The following contents are also included in the aspartyl dipeptide ester derivatives used for the solid sweetener composition of the present invention as a preferable derivative thereof.
[1] The derivative of the formula (2) described above, wherein R₃ is a hydroxyl group or a methoxy group, and R₄ and R₅ are a hydrogen atom.
[2] The derivative of the formula (2) described above, wherein R₁ is a hydroxyl group.
[3] The derivative of the formula (2) described above, wherein R₁ is a hydrogen atom.
[4] The derivative of the formula (2) described above, wherein R₂, R₆ and R, are a hydrogen atom.
[5] The above any derivative of the formula described above, wherein R₂ is a hydrogen atom, a hydroxyl group or a methyl group.

The derivatives described above included those in the form of salts, for example, edible salts form such as hydrochloride salts, sodium salts, potassium salts, ammonium salts, calcium salts and magnesium salts and so on.

For the particularly preferable aspartyl dipeptide ester derivative used for the solid sweetener composition of the present invention, 9 derivatives shown in the following table 1 can be enumerated. (All of R₈, R₉ and R₁₀ denote a hydrogen atom.)

**[Table 1]**

| Derivative No. | R₁ | R₂ | R₃ | R₄ | R₅ | R₆ | R₇ |
|---|---|---|---|---|---|---|---|
| 1 | H | OH | OCH₃ | H | H | H | H |
| 2 | H | H | OCH₃ | H | H | H | H |
| 3 | H | OH | OCH₃ | H | H | CH₃ | CH₃ |
| 4 | H | CH₃ | OH | H | H | CH₃ | CH₃ |
| 5 | H | H | OCH₃ | H | H | CH₃ | CH₃ |
| 6 | H | H | OH | H | H | CH₃ | CH₃ |
| 7 | OH | H | OCH₃ | H | H | H | H |
| 8 | H | CH₃ | OH | H | H | H | H |
| 9 | OH | H | OH | H | H | H | H |

As described above, only one or a mixture of plural of said derivatives can be used for the solid sweetener composition of the present invention. Furthermore, it can include another sweetener ingredient, within the scope in which the object of the present invention is not inhibited.

The "filler" in the solid sweetener composition of the present invention, means the ingredients which adjust the sweetness of the derivatives with a high intense sweetness, and comprises sugars, sugar alcohols, oligosaccharides, polysaccharides and so on, and further comprises those used as bulking agents, carriers and so on which are not tasty ingredients and ingredients used for carrier and so on.

As a solid filler, there is employed preferably at least one of the compounds contained in the group consisting of sugar, sugar alcohol, oligosaccharide and polysaccharide.

The sugar comprises sucrose (including derivative thereof), invert sugar, isomerized sugar, glucose, fructose, lactose, malt sugar, D-xylose and isomerized lactose, the sugar alcohol comprises maltitol (including reduced malt sugar syrup and so on), sorbitol, mannitol, erythritol, xylitol, lactitol (including reduced lactose and so on), paratinit, and reduced starch sugar (including hydrogenated starch syrup and so on). The oligosaccharide comprises fructooligosaccharide (including neosugar and so on), maltooligosaccharide (including linear oligosugar and so on), isomalto-oligosaccharide (including branched oligosugar and so on), galactooligosaccharide, soy been oligosaccharide and lactooligosaccharide, and further the polysaccharide comprises glucomannan, dietary fiber (including enzyme decomposition products of guar gum [galactomannan Hydrolysate and so on]), non-digestible dextrin (dextrin including dietary fiber), polydextrose and starch (including dextrin, soluble starch, modified starch and so on) and so on. Only one or a mixture of plural of said filler can be used for the solid sweetener composition of the present invention.

Incidentally, in said sucrose, derivatives of sucrose are included, and for example, sugar bound syrup (including coupling sugar, glucosylsucrose and so on), paratinose (including isomaltulose and so on) , trehalose and so on are cited therefore.

Furthermore, it can include other fillers (including carrier, bulking agent and so on), within a range in which the object of the present invention is not inhibited.

In the solid sweetener composition of the present invention, it may comprise at least a step of mixing said derivative in the form of a solution during manufacture. In such case, it is preferable to dissolve the derivative completely, and it is also acceptable to disperse the derivative homogeneously, or the intermediate state thereof may be acceptable (partial dissolution and remaining partial dispersion).

Preferably, the aimed solid sweetener composition of the present invention can be obtained by mixing a total amount of composition homogeneously in the form of a solution, and drying the same in case of necessity. As the solvent therefor, a solvent usable for drinking and eating which dissolves the derivative used for the solid sweetener composition of the present invention, and preferably a filler may be used. As the solvents only one kind or a mixture thereof is used. There are preferably enumerated water, alcohol such as ethanol, polyvinyl acetate, oils and fats, and so on. For a method for mixing the composition homogeneously, a method for homogeneous mixing in the liquid-solid system, or known or available methods as for mixing homogeneously in the liquid system, can be adopted. The method of removing the solvent in case of necessity is required, and various available drying methods can be employed.

In the solid sweetener composition of the present invention, a sweetener (tabletop use or others) , and the food in the form of solid containing further components necessary for foods in the composition in addition thereto and obtained by these methods, are also contained in the present invention. As such examples, powdered juice, powdered cocoa, powdered cola, instant coffee, black tea and so on, chocolate, chewing gum, health food, medicine and so on are included.

And, food and drink (a cola drink) which is obtained or can be obtained by using said solid sweetener composition in the present invention, other products obtained as the product in the present invention (solid sweetener compositions) such as juice obtained by dissolving the powdered juice in water, and the thus sweetened products by the product in the present invention (bread with the sweetener of the present invention used, cakes with the sweetener or chocolate of the present invention used, and food with the product of the present invention used as a topping such as a yogurt and so on) are included in the solid sweetener composition of the present invention or the use thereof.

On the other hand, as a result of further studies to solve one of the problems above to be solved by the present invention, that is to provide and obtain the sweetener composition satisfied for not only the quality of end product, but also process control and operational efficiency during the production and delivery, the present inventors succeeded in the development of a stable liquid sweetener composition which comprises said aspartyl dipeptide ester derivative with a high intense sweetness in an edible medium such as water and alcohol and so on. The present inventor has found a solution in which said aspartyl dipeptide ester derivative has been dissolved using an edible medium, and preferably a suspension in which said aspartyl dipeptide ester derivative has been stably dissolved and stably dispersed. These findings have led to the accomplishment of the invention "a liquid sweetener composition" in which an another embodiment of the present invention.

That is, the present invention lies in a liquid sweetener composition comprising a solution which has dissolved at least one of aspartyl dipeptide ester derivatives (which may be in the salt form) represented by the following general formula (2), more preferably the following general formula (1), in an edible medium such as water and alcohol.

Said aspartyl dipeptide ester derivative used for the liquid sweetener composition in the present invention includes those in the form of salts, and only one derivative or a mixture of not less than two derivatives is used. 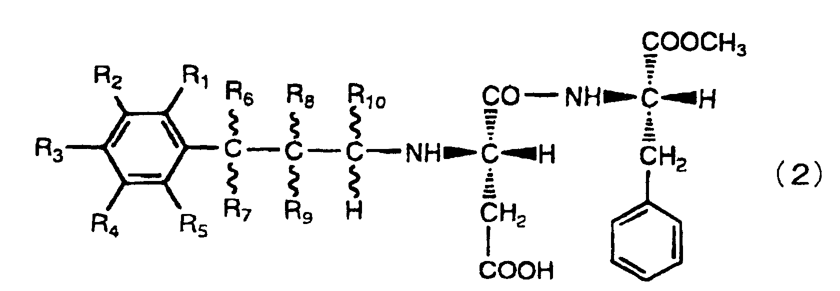 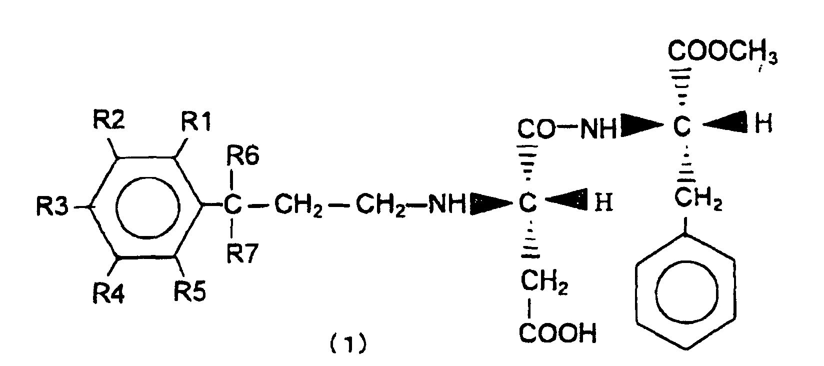 wherein in the above formulae R₁, R₂, R₃, R₄ and R₅ are independent from each other, and each denotes any one selected from the group consisting of a hydrogen atom, a hydroxyl group, an alkoxy group having 1 to 3 carbon atoms (a methoxy, an ethoxy and an n-propoxy group and so on) , an alkyl group having 1 to 3 carbon atoms (methyl, ethyl, n-propyl and so on) and a hydroxyalkyloxy group having 2 or 3 carbon atoms (O(CH₂)₂OH, OCH₂CH(OH)CH₃ and so on) , and herein as for R₁ and R₂, or R₂ and R₃, the respective two symbols (R₁ and R₂, or R₂ and R₃), combined together with each other may form a methylene dioxy group (OCH₂O).

R₆, R₇, R₈, R₉ and R₁₀ are independent from each other, and each denotes a hydrogen atom or an alkyl group having 1 to 3 carbon atoms (methyl, ethyl, isopropyl and so on), respectively, herein any two substituents optionally selected from the group consisting of R₆, R₇, R₈, R₉ and R₁₀, combined together with each other, may form an alkylene group having 1 to 5 carbon atoms (CH₂, CH₂CH₂, CH₂CH₂CH₂ and so on).

When R₆ and R₇, or R₈ and R₉ denote different substituents with each other, or R₁₀ denotes an substituent except for a hydrogen atom, the configuration of the carbon atom to which these substituents (R₆ and R₇, R₈ and R₉ or R₁₀) are linked, has no restriction, and may be any one of (R), (S) and (RS) or mixture thereof, for example. In addition, wiggly lines described as the bond of R₆ to R₁₀, and an hydrogen atom with a carbon atom in said general formula (2), means that the direction of the bond is free (is not specified).

However, the derivative in which R₆ denotes a hydrogen atom or a methyl group and R₁, R₂, R₃, R₄, R₅, R₇, R₈, R₉ and R₁₀ denote a hydrogen atom concurrently is excluded. The derivative in which R₂ or R₄ denote a methoxy group, R₃ denotes a hydroxyl group, R₁₀ denotes a hydrogen atom or a methyl group, and R₁, R₄, R₅, R₆, R₇, R₈, and R₉ denote a hydrogen atom concurrently is also excluded.

As an aspartyl dipeptide ester derivative used for the liquid sweetener composition of the present invention, the derivative represented by said formula wherein all of R₈, R₉ and R₁₀ denote a hydrogen atom is more preferable.

The sweetness intensity of the aspartyl dipeptide ester derivative used for the liquid sweetener composition of the present invention is preferably more than 4,000 times that of sucrose.

The following contents are also included in the aspartyl dipeptide ester derivatives used for the liquid sweetener composition of the present invention as a preferable derivative thereof.
[1] The derivative of the formula (2) described above, wherein R₃ is a hydroxyl group or a methoxy group, and R₄ and R₅ are a hydrogen atom.
[2] The derivative of the formula (2) described above, wherein R₁ is a hydroxyl group.
[3] The derivative of the formula (2) described above, wherein R₁ is a hydrogen atom.
[4] The derivative of the formula (2) described above, wherein R₂, R₆ and R₇ are a hydrogen atom.
[5] The above any derivative of the formula described above, wherein R₂ is a hydrogen atom, a hydroxyl group or a methyl group.

The derivatives described above include those in the form of salts, for example, edible salts form such as hydrochloride salts, sodium salts, potassium salts, ammonium salts, calcium salts and magnesium salts and so on.

For the particularly preferable aspartyl dipeptide ester derivative used for the liquid sweetener composition of the present invention, 9 derivatives shown in the following table 2 can be enumerated. (All of R₈, R₉ and R₁₀ denote a hydrogen atom.)

**[Table 2]**

| Derivative No. | R₁ | R₂ | R₃ | R₄ | R₅ | R₆ | R₇ |
|---|---|---|---|---|---|---|---|
| 1 | H | OH | OCH₃ | H | H | H | H |
| 2 | H | H | OCH₃ | H | H | H | H |
| 3 | H | OH | OCH₃ | H | H | CH₃ | CH₃ |
| 4 | H | CH₃ | OH | H | H | CH₃ | CH₃ |
| 5 | H | H | OCH₃ | H | H | CH₃ | CH₃ |
| 6 | H | H | OH | H | H | CH₃ | CH₃ |
| 7 | OH | H | OCH₃ | H | H | H | H |
| 8 | H | CH₃ | OH | H | H | H | H |
| 9 | OH | H | OH | H | H | H | H |

The edible medium (water, alcohol and so on) can include a stabilizer (bulking agent), a thickening agent, a filler and so on. These are preferably used as a medium for suspension.

At least one of the compounds contained in the group consisting of sugar, sugar alcohol, and oligosaccharide can be added as a stabilizer. As a result, a suspension having a high solubility, a high dispersibility and a high stability in terms of said derivative can be prepared.

There can be enumerated for a preferable stabilizer, sucrose (including derivative thereof), invert sugar, isomerized sugar, glucose, fructose, lactose, malt sugar, D-xylose and isomerized lactose regarding the sugar; maltitol, sorbitol, mannitol, erythritol, xylitol, lactitol, paratinit, and reduced starch syrup and hydrogenated starch hydrolysate regarding the sugar alcohol, and fructooligosaccharide, maltooligoosaccharide, isomaltooligosaccharide, galactooligosaccharide, soy been oligosaccharide and lactooligosaccharide regarding the oligosaccharide. Besides, as the derivative of sucrose, there are enumerated sugar bound syrup (including coupling sugar, glucosylsucrose and so on), palatinose (including isomaltulose and so on) and trehalose and so on.

As described above, it is preferable to use a liquid medium such as water, alcohol, and a mixed solvent containing any one of water and alcohol and so on as the edible medium, and the stabilizer as described above may be included. A liquid solution can be prepared in the form of suspension, especially preferably homogeneous suspension, which contains at least one of said derivatives in the higher concentration than that in the solubility thereof in the liquid medium.

The sweetener composition in the form of stable suspension can be obtained by mixing the suspension which contains at least one of said derivatives in the higher concentration than that in the solubility thereof in the liquid medium, with a liquid medium. For the mixing method in such mixture, the vacuum mixing method is preferably used.

The liquid sweetener composition in the present invention comprises the matter in the form of a food and drink such as a sweetener, a drink, a frozen dessert, a syrup and so on, a pharmaceutical product (medicine) and so on.

In addition, the present invention comprises food and drink such as a drink, a frozen dessert, a desert and so on, pharmaceutical product, oral cosmetics and another sweetened product and so on, which has used the above such liquid sweetener composition.

### Preferred Embodiments

Hereinafter, the preferred embodiments in the present invention are explained, and however they are explained at first for the solid sweetener composition of the present invention and then for the liquid sweetener composition of the present invention.

### (The solid sweetener composition of the present invention)

As the aspartyl dipeptide ester derivatives represented by said general formula (2), which are used for the solid sweetener composition in the present invention, particularly said 9 derivatives (referred to as "derivative 1" to "derivative 9" respectively) are preferable in the point of a high intense sweetness. The solid sweetener composition of the present invention, therefore, is explained mainly on these derivatives, however, the solid sweetener composition of the present invention is not limited to the use of these derivatives.

The aspartyl dipeptide ester derivatives used for the solid sweetener composition of the present invention can be easily synthesized by alkylating Aspartame reductively with 3-phenylpropionaldehyde derivative, cinnamaldehyde derivative or (2-phenylethyl) alkyl ketone derivative having various substituents on the phenyl group and one or two alkyl substituents on the main chain, and a reducing agent (e.g., hydrogen/palladium carbon catalyst). Alternatively, they can be obtained by the process comprising alkylating the Aspartame derivative having a protecting group for the carboxyl group at the β position (for example, β-o-benzyl-α-L-aspartyl-L-amino acid methyl ester), which can be obtained by ordinary peptide synthesis method (Izumiya et al., Fundamentals and experiments of peptide synthesis: Maruzen, published on 1985.1.20), reductively with the 3-phenylpropionaldehyde derivative, cinnamaldehyde derivative or (2-phenylethyl) alkyl ketone derivative described above, and a reducing agent (e.g., NaB(OAc)₃H) (A.F.Abdel-Magid et al., Tetrahedron Letters, 31, 5595 (1990)), and then removing the protecting group, or by saturating the unsaturated bond with a reducing agent, if needed. Instead of said 3-phenylpropionaldehyde derivative, cinnamaldehyde derivative or (2-phenylethyl) alkyl ketone derivative, an acetal or ketal derivative thereof or so on can be certainly used as an aldehyde or ketone component for the reductive alkylation.

These derivative can be easily produced by known peptide synthesis method as shown above, or according to the production examples on the derivatives 1 to 9 as shown in the Examples described later.

There is no particular restriction to the mixing ratio of the aspartyl dipeptide ester derivatives and the solid filler used for the solid sweetener composition of the present invention.

With regard to the content, however, the aspartyl dipeptide ester derivatives used for the solid sweetener composition of the present invention can be used in the range of preferably 2 ppm (by weight) to 95% (by weight) or so in the ratio of said aspartyl dipeptide ester derivative(s) (one or two derivatives or more) to a total amount of said aspartyl dipeptide ester derivative (s) and said filler. Also said derivative (s) can be used preferably 0.2 ppm (by weight) to 95% (by weight) or so in the ratio of said aspartyl dipeptide ester derivative(s) to a total amount of said aspartyl dipeptide ester derivative(s) and said filler.

When the composition is mixed, the aspartyl dipeptide ester derivative used for the solid sweetener composition of the present invention, is mixed at least with the component (s) for the composition including the filler in the form of a solution, and the total composition can be mixed homogeneously in the form of a solution.

The solid sweetener composition in the present invention can be used as a sweetener, for example, a sweetener for tabletop use (a sweetener placed on the table) . In this case, it is sufficient for the sweetener to contain at least one of said aspartyl dipeptide ester derivatives and one of the solid fillers.

Furthermore, the sweetener can be mixed with another sweetener ingredient(s) (another sweetener with a high intense sweetness such as Aspartame, and the third and fourth sweetener ingredients such as sugar, sugar alcohol and so on). A necessary ingredient other than the sweetener ingredient, such as salt like sodium chloride can be also mixed therewith.

When the solid sweetener composition in the present invention is used for a sweetener, there can be used a carrier, a bulking agent, and so on necessary for a sweetener other than said filler (included in the filler used for the solid sweetener composition in the present invention). In this case, a carrier, a bulking agent and so on for the sweeteners which have been known or used so far, can be used, for example.

The solid sweetener composition of the present invention comprises a composition consisting essentially of a sweet substance and a filler, such as a sweetener, and a food consisting essentially of a sweet substance and a filler such as a fondant-like food. Moreover, the food composition having a sweet taste by mixing various ingredients which are necessary for food other than said filler, said sweetener ingredient and so on in addition thereto, can be produced, and this is also included in the present invention. In said sweetener composition, are mixed therewith the solid ingredient(s) necessary for each object other than the sweetener ingredient and the filler ingredient in addition thereto. An ingredient which tastes a fruit taste for the powdered juice, flavor ingredients necessary for candy and jelly, an ingredient for tablet candy (that outer side is prepared separately) therefor, nutritious ingredients for nutritional supplements, pharmaceutical active ingredient(s) for pharmaceutical products, coffee ingredients for powdered coffee, dairy ingredients for powdered dairy products, dentifrice ingredients for tooth paste and tooth powder and so on are mixed.

Specifically, a tabletop use of sweetener (coating the surface of the fine crystals in powder filler), powdered cocoa, powdered cola, powdered coffee (spray dried product) , instant coffee, health care food (freeze dried product) and powdered juice (concentrated dried product) as a powder-type product, medicines as a granular-type product, powdered flavor seasoning as a granulated product, and chocolate, chewing gum, fondant-like food as an another solid product are enumerated.

There is not any special problem (difficulty) to produce the product in the present invention (the solid sweetener composition), and for example, by making use of a mixed dispersion method using a solvent. It can be performed some preferable methods are briefly explained as follows.
1. The composition is produced by drying a solution of the composition in which the composition ingredients are dissolved homogeneously.
2. The composition is solidified by any one of the methods consisting of condensation drying such as Aspartame-containing sugar (refer to Japanese Patent Kokai Publication JP-A-63-146768 and so on), spray drying such as the composition for imparting sweetness (refer to Japanese Patent Kokai Publication JP-A-58-20588 and so on), freeze drying such as instant coffee (refer to Japanese Patent Kokai Publication JP-A-59-45849 and so on), extrusion granulation such as low-calorie sweetener (refer to Japanese Patent Kokai Publication JP-A-1-206969 and so on), and absorption to forming sugar such as solid sweetener (refer to Japanese Patent Kokai Publication JP-A-58-36368 and so on).
   When said solution is used as a solvent which constitutes the solution , any one of water, alcohol such as ethanol, and a homogeneously mixed solvent comprising at least any one of the both, is preferable. That is, a single water solvent, or a single alcohol solvent is used frequently, but a mixed solvent which comprises at least one of water and alcohol such as a mixed solvent of water and alcohol and so on can be also used.
3. The solution of said aspartyl dipeptide ester derivative(s) is coated on the surface of the filler homogeneously, for example, according to the production of the composition containing Erythritol (refer to Japanese Patent Kokai Publication JP-A-4-335870 and so on).
4. As a solvent which constitutes the solution, water, alcohol such as ethanol or a mixed solvent comprising at least one of water and alcohol is used, and said solution is spread by spraying on the surface of the fine crystals of said powder filler for mixture. For example, a low-calorie sweetener composition in the form of fine crystals in appearance (refer to Japanese Patent Kokai Publication JP-A-1-95741 and so on) is cited therefor.

On the other hand, by using the solid sweetener composition in the present invention as a sweetener (the product of the present invention), for various products such as food and drink which are in need of giving sweet taste, for example, a fruit juice drink, cola drink, a frozen dessert, an ice cream, an ice lolly, a bread, a cake and so on, a sanitary product, cosmetics (including an oral composition such a tooth paste and powder), a medicine, a product for an animal other than human, and so on, the each end product can be produced, and food and drink and so on which can be obtained by using in such manner the solid sweetener composition in the present invention as said sweetener which is a product of the present invention, or another product of the present invention (chocolate and so on) are also contained in the present invention.

For example, food and drink and so on which are obtained by using the solid sweetener composition in the present invention directly (use as an intrinsic object), such as the juice having a homogeneously dispersed sweetness obtained by dissolving in a water the powdered juice (a solid sweetener composition) which is a product in the present invention are contained naturally in the present invention.

### (The liquid sweetener composition of the present invention)

Hereafter, the liquid sweetener composition in the present invention is explained.

With regard to the aspartyl dipeptide ester derivatives represented by said general formula (2), particularly said general formula (1), which are used for the liquid sweetener composition in the present invention, particularly the aforementioned 9 derivatives (referred to as "derivative 1" to "derivative 9" respectively) are preferable in the point of a high intense sweetness. The liquid sweetener composition of the present invention, therefore, is explained mainly on these derivatives, however, the liquid sweetener composition of the present invention is not limited to the use of these derivatives.

The aspartyl dipeptide ester derivatives used for the liquid sweetener composition in the present invention can be easily synthesized by alkylating Aspartame reductively with 3-phenylpropionaldehyde derivative, cinnamaldehyde derivative or (2-phenylethyl) alkyl ketone derivative having various substituents on the phenyl group and one or two alkyl substituents on the main chain, and a reducing agent (e.g., hydrogen/palladium carbon catalyst). Alternatively, they can be obtained by the process comprising alkylating the Aspartame derivative having a protecting group for the carboxyl group at the β position (for example, β-o-benzyl-α-L-aspartyl-L-amino acid methyl ester), which can be obtained by ordinary peptide synthesis method (Izumiya et al., Fundamentals and experiments of peptide synthesis: Maruzen, published on 1985.1.20), reductively with the 3-phenylpropionaldehyde derivative, cinnamaldehyde derivative or (2-phenylethyl) alkyl ketone derivative described above, and a reducing agent (e.g., NaB(OAc)₃H) (A.F.Abdel-Magid et al., Tetrahedron Letters, 31, 5595 (1990)), and then removing the protecting group, or by saturating the unsaturated bond with a reducing agent, if needed. Instead of said 3-phenylpropionaldehyde derivative, cinnamaldehyde derivative or (2-phenylethyl) alkyl ketone derivative, an acetal or ketal derivative thereof or so on can be certainly used as an aldehyde or ketone component for the reductive alkylation.

These derivative can be easily produced by known peptide synthesis method as shown above, or according to the production examples on the derivatives 1 to 9 as shown in the Examples described later.

The sugar, sugar alcohol and oligosaccharide used for the liquid sweetener composition of the present invention as a stabilizer (which may be hereinafter generically referred to as " sugar and so on used for the liquid sweetener composition of the present invention") are explained.

With regard to the sugar, among sugars any sugar which has a sweet taste and is soluble in water, is preferably used. For example, it comprises sucrose (including derivative thereof), invert sugar, isomerized sugar, glucose, fructose, lactose, malt sugar, D-xylose and isomerized lactose. The derivative of sucrose comprises, for example, sugar bound syrup (including coupling sugar, glucosylsucrose and so on) , paratinose (including isomaltulose and so on) and trehalose and so on.

The term "sugar alcohol" means a reduced sugar, and the term "oligosaccaride" means a polysaccharide which has several basic frames of monosaccharide such as glucose and fructose. With regard to the sugar alcohol, there are enumerated maltitol, sorbitol, mannitol, erythritol, xylitol, lactitol, palatinit, reduced starch sugar and hydrogenated starch hydrolysate. With regard to the oligosaccharide, there are enumerated fructooligosaccharide, maltooligosaccharide, isomaltooligosaccharide, galactooligosaccharide, soy been oligosaccharide and lactooligosaccharide.

When these compounds are used as a stabilizer in the liquid sweetener composition of the present invention, either one or plural compounds thereof can be used.

Among the above described compounds, in view of improving the stability (in high solubility and high dispersibility) of the aspartyl dipeptide ester derivatives used for the liquid sweetener composition of the present invention, it is preferable to mix for use isomerized sugar, sugar alcohol such as sorbitol, hydrogenated starch hydrolysate and coupling sugar and so on.

By using these stabilizers, said derivative used for the liquid sweetener composition of the present invention can be included. There is obtained the liquid composition with a high intense sweetness of which the sweetness is enriched by the addition of the sugar and so on used for the liquid sweetener composition of the present invention, and at the same time said derivative is also held with extreme stability even in the liquid system . When it is used, particularly in the suspension, the crystals of said derivative are dispersed in the liquid solution homogeneously for suspension, and thereby the good effects on dispersibility and solubility in the edible medium such as water are generated. As a result, the product of the present invention (the liquid sweetener composition) can be used in the form of sweetener or food and drink, and it can also provide a highly practical sweetener for the raw material of drink, desert, frozen dessert and so on.

Said derivatives used for the liquid sweetener composition of the present invention, have generally low solubility in water (1 to 1000mg/100ml), however, their small amount of solubility assures sufficiently a intense sweetness due to their high intense sweetness. They can also be used as a suspension preferably which contains said derivatives in a concentration higher than their solubility in the medium. When the viscosity of the suspension medium (dispersion medium) in the production of the suspension is low, the heterogeneous sedimentation is sometimes observed, however, in this case the suspension can be dispersed homogeneously by the agitation such as shaking in use. In this case, in order to keep the suspension always homogeneous, it is preferable to select and use the suspension medium which has at least such a viscosity so as to keep a stability of the suspension of said derivative (more preferably, the solution containing the sugar and so on used for the liquid sweetener composition of the present invention as a stabilizer in the edible medium such as water), or to add further a gum substance such as xanthan gum, guar gum and so on, a viscosity-improving stabilizer such as polysaccharide and a specific gravity-increasing component to be added to improve much more the stability of dispersion and suspension of said derivative in the suspension medium.

With regard to an amount for addition of said derivative(s) (one kind or more) to the suspension medium, it is preferable to use the amount thereof such that at least one part of said derivative can be maintained in the insoluble state stably.

The lower limit of the amount for addition of said derivative to produce the suspension, is an amount thereof necessary sufficient for super saturation of said derivative under a storage or room temperature, and the higher limit thereof is an amount of said derivative required for the target intensity of sweetness. When the amount of said derivative required for the target intensity of sweetness is less than the amount of saturation therefor, it is of course in the solution form therewith dissolved which can be obviously used as a liquid sweetener composition of the present invention.

When the stabilizer (trituration) which can be used by adding to the suspension medium, one or more thereof can be used in a single or combination form. Further, other seasoning ingredient(s) (such as sodium L-glutamate, tasty (gustatory) substance like 5'-nucleotide, sweetening substance like steviosides, saccharine and so on, organic acid, amino acid, peptides, extracts and so on), flavor, spice, colarant, inorganic substance like calcium and magnesium, vitamin, and lipid and so on may be used at the same time. When fats and oils are used at the same time, they can be provided as a emulsion of o/w or w/o type.

The liquid sweetener composition of the present invention is produced without particular problem, for example, also by simply mixing directory said derivative (one or more kinds) for mixture to be able to make it in the form of solution or suspension (slurry like). It can also be produced by heating the suspension medium used (preferably the solution containing sugar and so on as a stabilizer in the water ) , adding and mixing said derivative with the medium and then cooling the mixture. The following methods are preferably used to disperse said derivative homogeneously without hugging air bubbles.

The suspension (slurry) containing said derivative and water (and/or a part of stabilizer) is prepared, and then mixed with stabilizer (when a part thereof has been already used, all of remainder). In this process, it is preferable that the suspension (slurry) is prepared by directly mixing (homogenating) said derivative with a part of said stabilizer previously, or mixing (homogenating) said derivative with water at first, and then mixing (homogenating) the same with a part of stabilizer followed by the following processes. These mixtures are further mixed with a lot of remainder of stabilizer, preferably vacuum mixing, under preventing the mixture from forming air bubbles, and thereby the liquid sweetener composition in which the particles of said derivative are homogeneously dispersed, can be produced.

In the present invention, the forms of the liquid sweetener composition include those of liquid (homogeneous solution or homogeneous dispersed solution), paste-like, and fluid or semi-fluid of soft or hard cream-like and so on. Such those all forms are included in the present invention. For example, in case of the paste-like form, or cream-like form, it is superior in the operation of mixing with the highly viscous raw material(s), and thus preferable for producing the frozen dessert and so on.

Those liquid sweetener compositions of the present invention are superior in the stability for storage of said derivative (The derivative does not decompose by itself and keeps the high solubility and high dispersibility for long time.), and therefore, the liquid composition of sweetener with a high intense sweetness (the liquid sweetener composition with a high intense sweetness) can be obtained without loss of sweetness. In addition, because the use of stabilizer makes it possible to improve the dispersibility and solubility in water extremely compared with the use of said derivative alone, said sweetener composition is expected for wide and various uses. It can be used widely for drinks, sherbets, syrups and venders and so on.

In the liquid sweetener composition of the present invention, there is no particular restriction to the mixed composition of said aspartyl dipeptide ester derivative(s) and the sugar and so on used for the liquid sweetener composition of the present invention as a stabilizer.

The use of said sugar and so on makes it stable, and also improves or adjusts the characteristics of sweetness by selecting the preferable sugar and so on.

In solubility and dispersibility, it can be effective for obtaining the more stability of said composition, to use viscosity-improvers, fillers or surfactants furthermore.

Moreover, in the liquid sweetener composition, another type of sweetener ingredient (the third and fourth sweetener ingredients: another sweetener with a high intense sweetness such as Aspartame) can be mixed therewith. It can be also mixed with a necessary ingredient other than the sweetener ingredient, such as salt like sodium chloride.

The liquid sweetener composition in the present invention can be used typically for a sweetener (a product of the present invention). In this case, at least one of said derivatives (may be in the salt form) and at least one of the stabilizers can be included in the sweetener composition wherein the derivative(s) may be dissolved stably in the edible medium. Particularly, in case of suspension, said derivative which is insoluble in part, may be preferably dispersed homogeneously. It is preferable to use the edible medium such as water and so on containing such stabilizer(s), as a medium for solution or suspension in the present invention.

When the liquid sweetener composition of the present invention is used as a sweetener or a food and drink, there can be used a carrier, a thickening agent, a bulking agent, and/or a filler for sweeteners, where necessary to improve the more stability as described above. In this case, for example, a carrier, a thickening agent, a bulking agent, and a filler and so on for the sweeteners which has been known or used so far, can be used.

As such carrier and so on, there can be used aforementioned sugar and so on illustrated as a stabilizer, or besides others which have been used or can be used so far as a carrier for a sweetener.

The product of the present invention can be produced in the form of a sweetener, and besides the another form, that is the form of food and drink such as a frozen dessert by using the components and fillers necessary for food and drink.

In addition, the liquid sweetener composition of the present invention, can be used as a sweetener for various products, that is a product like food and drink, for example, a confectionery (a frozen dessert, a jelly, a cake, a candy), bread, chewing gum, a sanitary product, cosmetics (including an oral composition such a tooth paste and powder), a medicine and a product for an animal other than human which are in need of sweet taste imparted. Both in the form of such sweetened products, and in the method for imparting a sweetness to a product which is in need of sweetness, the liquid sweetener composition in the present invention can be used, which are naturally included in the present invention. With regard to the method for using them, any method which is known as a method used in case of using a sweetener for a sweetener or a method for imparting a sweetness can be adopted.

By using the liquid sweetener composition in the present invention, there is provided a sweetener of homogeneous sweetness which can be used stably for a long time with saving a space, and excellent in a solubility and handling, without a problem of scattering of fine particle powder.

### Examples

Hereinafter, the present invention is explained in more detail by reference to Examples, Comparable Examples and further Production Examples of the aspartyl dipeptide ester derivatives which are used for the present invention.

### (Example 1) Measurement of the magnification of sweetness intensity

### [Method for determining the magnification of sweetness intensity]

An aqueous solution was prepared by diluting derivative 2 to be PSE 10% concentration (15.5 mg/1000 ml = 10/6500 g/100 ml), assuming that the intensity of sweetness of derivative 2 was 6500 times that of sucrose. Separately, aqueous sucrose solutions having sucrose concentrations of (a)6.94%, (b)8.33%, (c)10%, (d)12%, and (e)14.4% were prepared. The sensory evaluation was performed by determining which sucrose solution was closest to the solution of derivative 2 in the sweetness intensity. The result of calculation of the average of points of 20 panelists was 2.25 point.

The sweetness intensity of the solution of derivative 2 was 8.75% according to the following equation: (10.0-8.33) x 0.25+8.33=8.75. Therefore, the magnification of sweetness of derivative 2 was 5600 (=8.75/0.00155) times that of sucrose. According to the same experiment, the magnification of sweetness intensity of derivative 1 was 22600 times that of sucrose. Further, the magnification of sweetness intensity of other derivatives (3 to 9) can be determined in the same manner. And the magnification of sweetness intensity in a cola drink can be also determined by the same method compared to the control solution of cola drink containing 10% sucrose.

Incidentally, the composition of cola drink is as follows.

| | |
|---|---|
| Citric acid (crystal) | 0.25g/1000ml |
| Sodium citrate | 0.10g/1000ml |
| 85% Phosphoric acid | 0.3g/1000ml |
| Cola base | 2ml/1000ml |
| Cola essence | 1ml/1000ml |
| Sweetener (sample) | Prescribed amount |

Incidentally, as for the concentration of the references, the sucrose concentrations of previous (a) to (e) were used.

### [Results]

The magnifications of sweetness intensity of the derivatives relative to that of sucrose measured as described above were shown as follows.

| Samples | In aqueous solution | In cola drink |
|---|---|---|
| Derivative 1 | 22600 | 22600 |
| Derivative 2 | 5500 | 4900 |
| Derivative 3 | 42400 | 37000 |
| Derivative 4 | 43500 | 29600 |
| Derivative 5 | 8400 | 8000 |
| Derivative 6 | 14900 | 14000 |
| Derivative 7 | 11100 | 10600 |
| Derivative 8 | 18200 | 15800 |
| Derivative 9 | 8000 | 7500 |

### (Example 2) Production of orange juice powder

The following ingredients were dissolved in 1000g of water and spray dried to produce orange juice powder.

| Components | Amount (g) |
|---|---|
| Derivative 6 | 0.023 |
| Orange juice micron D-50^{*1} | 31.6 |
| Anhydrous citric acid | 4.0 |
| Malic acid | 8.0 |
| Sodium citrate | 2.8 |
| Orange micronZD-0568^{*2} | 4.8 |
| Orange color baseW-6540^{*3} | 1.6 |
| Fruit micronCL-2068^{*4} | 4.0 |
| Vitamin C | 2.0 |
| Powdered maltitol | 41.177 |
| Total | 100 |

| | |
|---|---|
| *1-4: Takasago Koryo, Co. Ltd. | |

5 g of the powder thus produced are dissolved in 150 ml of water to make an orange juice of about PSE 12% for drinking. Here, the magnification of sweetness intensity is calculated on the basis of derivative 6: 14000 times, solid component of concentrated fruit juice of orange: 1, maltitole: 0.75.

5g of the powder produced were collected from 20 points at random, and each sample was dissolved in 150 ml of water to compare the sweetness intensity. There was no significant difference among the respective samples. The sweet taste of the solutions was equivalent to that of about PSE 12%, had been mixed and dispersed homogeneously (n=20).

On the other hand, when each 5g of the sample was collected from 20 points of the mixture obtained only by mixing said components with said composition, and evaluated as described above, there was a significant difference in the sweetness among the respective samples, and confirmed to be heterogeneous state.

As described above, the sweetness of the sample prepared by dissolving previously and then spray drying, tasted the dispersed and preferable sweetness compared with the case of mixing powders each other. As is understood by example 1 described above, it is thought that the sweetness of the sample which is prepared by just sprinkling the powder or mixing the powders each other, is not dispersed homogeneously, because they taste a high intense sweetness and the amount of use is extremely small.

### (Example 3) Production of granular products

The sweetener composition which is easy for handling, is provided by improving the property of the aspartyl dipeptide ester derivative which is a sweetener with a high intense sweetness, and used for the present invention.

The crude powder of said derivative is generally fine, needle-like crystals, large specific volume and easy to scatter. Further, it is not dispersed or dissolved well in water. Thus, if the crude powder is dissolved in water, coagulates (insoluble aggregations) are formed, and it is difficult to dissolve in water. In addition, as the intense sweetness of said derivative is not less than 4000 times to 50000 times that of sucrose, it is necessary to measure the very small amount precisely and to mix homogeneously, when it is used as a crude powder.

However, the granular product having an improved physical property such as the solubility and dispersibility, and in which said derivative is mixed homogeneously without scattering, could be produced by the following methods.

### (Ex. 1)

87 weight parts of anhydrous lactose and 8 weight parts of dextrin were mixed, arid then the solution dissolved 0.2 weight parts of derivative 2 (the magnification of sweetness intensity is 5000 times) in 15 weight parts of water was added to the mixture and mixed. And the mixture was granulated by extruding, and dried to obtain the granular product. The magnification of sweetness intensity: approximately 10 times.

The results of the measurement of the physical property is as follows.
Crude specific volume: 1.47 cc/g, Fine specific volume: 1.46 cc/g, Under 16 mesh to over 80 mesh:80%, Rate of dissolving: approximately 35 seconds in hot water (40°C), approximately 26 seconds in cold water (10°C), Dispersibility: rapid dispersion without making insoluble coagulates, Fluidity: good.
*1: As for the rate of dissolving, the distilled water was poured in the 500 ml beaker, 1 g of sample was added to the beaker with gentle mixing by magnetic stirring, and the time of dissolving was measured.

And, when the rate of dissolving of the crude derivative 2 was measured in the same way, the insoluble coagulates of crude derivative 2 were formed even in the hot water, and did not reach the complete dissolution after 5 minutes.

### (Ex. 2)

90 weight parts of dextrin^{*1}, 5 weight parts of dextrin^{*2} and 12 weight parts of the solution dissolved 0.044 weight parts of derivative 1 (the magnification of sweetness intensity is 22600 times) in water, were mixed.
*1:Amicol H (Nichiden Kagaku);
*2:Amicol No.1 (Nichiden Kagaku).

According to the above mixing, the granular product was obtained by fluid granulation. The magnification of sweetness intensity: approximately 10 times.

The rate of dissolving and so on were measured by the same methods as described in Ex. 1. As the results, Crude specific volume: 3.48 cc/g, Fine specific volume: 2.87 cc/g, Fine granule: approximately 260 µm, Rate of dissolving^{*3}: approximately 15 to 20 seconds in hot water (40°C), approximately 60 seconds in cold water (10°C), Dispersibility: rapid dispersion without making insoluble coagulates, Fluidity: good.
*3: The method of measurement is same as described above.

### (Example 4) Production of sweetener for tabletop use

The sweetener granule containing the following ingredients was produced by flow granulation method. At the time, derivative 1 was dissolved in the water added. The condition of the granulation is as follows.
Type of coating machine:VG-1200, Feed volume:260 kg, Rotation frequency of main shaft:50 rpm, Rotation frequency of granulation shaft:900 rpm, Ratio of water added:1.0%, Mixing time:5 minutes, Temperature of hot air:80°C, Temperature of exhaust air: stop at 40°C, Particle size: ca. 700 µm, fine powder ratio (250 µm pass):2.5%.

| Components | Weight (Kg) | Composition (%) |
|---|---|---|
| Derivative 1 | 0.21 | 0.031 |
| Aspartame | 1.38 | 0.206 |
| Erythritol | 666.67 | 99.469 |
| Flavor | 1.97 | 0.294 |
| Total | 670.23 | 100 |

When 0.94 g of the sweetener produced were added to 140 ml (volume for the standard coffee cup) of coffee solution, the sweetness intensity of said coffee solution is equivalent to that of PSE 5%. The sweetness ratio of the sweetener is in derivative 1: Aspartame: Erythritol=4:0.5:0.5. Herein.the magnification of sweetness intensity of derivative 1 at PSE 4% was calculated as 18500 times, the magnification of sweetness intensity of Aspartame at PSE 0.5% was calculated as 360 times and the magnification of sweetness intensity of Erythritol at PSE 0.5% was calculated as 0.75 times.

When 5g of the sample obtained were collected from 20 points at random, and each sample was added to 140 ml of coffee solution to compare the sweetness intensity of the coffee solution, there was no significant difference among the respective samples, and the sweetness intensity of each of the solutions was equivalent to that of PSE 5% (n=20). The mixtures of the samples had been mixed and dispersed homogeneously.

On the other hand, when each 0.94 g of the sample were collected from 20 points of the mixture obtained only by mixing said components with said composition, and evaluated by adding to the coffee solution in the same way, there was a significant difference in the sweetness among the respective samples, and confirmed to be heterogeneous state (n=20).

### (Example 5) Production of fondant-like food

Fondant which is fine crystals of sucrose prepared by oversaturating sucrose solution and adding the impulse, is used as a decoration of cakes and sugar coat of Japanese confectionary. As the fondant is constituted by the particular shape which is enclosed by the syrup around the fine crystal of sucrose, it is confirmed that such a shape can not be formed when the sweetener composition of the aspartyl dipeptide ester derivative used in the present invention is used instead of sucrose.

On the other hand, the fondant-like foods comprising glucose, said derivatives and water at the ratio of 100:0:10 to 100:1:20 by weight, or glucose, lactose said derivatives and water at the ratio of 90:10:0:10 to 10:90:1:20 by weight, were produced.

### (Ex.1) Use of derivative 2 (as the magnification of sweetness intensity is 5000 times)

Glucose (100g), derivative 2 (0.04g) and water (20g) were mixed previously, heated for 18 minutes. After the temperature of the mixture was reached at 115°C, the mixture was cooled to 60°C and mixed to produce the fondant-like food. The magnification of sweetness intensity of this fondant-like food is approximately 2.2 times that of sucrose (when the magnification of sweetness intensity of glucose is 0.6).

### (Ex.2) Use of derivative 1 (as the magnification of sweetness intensity is 22600 times)

Glucose (50g), lactose (50g) and water (20g) were mixed, heated to 115°C, and cooled to 90°C. After that, derivative 1 (0.0088g) was added to the mixture and mixed and stirred to produce the fondant-like food. The magnification of sweetness intensity of this fondant-like food is approximately 2 times that of sucrose (when the magnification of sweetness intensity of lactose is 0.2).

### (Example 6) Production of chocolates

The chocolate of the following composition was produced by the following ordinary method. With respect to derivative 8, however, it was added with flavor at the time of triturating and mixing .

| Components | Composition (g/100ml) |
|---|---|
| Derivative 8 | 0.0015 |
| Maltitol | 40 |
| Cacaomass | 27 |
| Cacao butter | 23 |
| Powdered milk with lipid | 10 |
| Lecithin | 0.4 |
| Flavor | 0.1 |

After mixing cacaomass, cacao butter, powdered milk, maltitol and lecithin previously, the mixture was refining, couching, tempering, packing, cooling and maturing, and then the chocolate was produced. The cariostatic, low-calorie, light-taste chocolate was obtained.

### (Example 7) Production of chewing gums

The components of the following composition 1 were added to the kneader heated at 120°C sequentially, and melt-mixed for 10 minutes. In this time, derivative 2 had been dissolved in polyvinyl acetate previously. Then, the components of the composition 2 were added to the kneader sequentially, and melt-mixed for 10 minutes in the same way. This mixture was used cooled down to room temperature, and then used as a gum base. By using this gum base, the components of the composition 3 were added to the kneader pre-heated at 75°C sequentially, and then heating was stopped. After mixing for 12 minutes and rolling by roller, chewing gum was produced.

### Composition 1:

| Components | Mixed amounts (%) |
|---|---|
| Derivative 2 | 0.0003 |
| Polyvinyl acetate | 40 |
| Monoglyceride | 3.5 |
| Polybutene | 3.5 |

### Composiion 2 :

| Components | Mixed amounts (%) |
|---|---|
| Talc | 17 |
| Gelton | 10 |
| Wax | 6 |
| Ester gum | 20 |

### Composition 3:

| Components | Mixed amounts (%) |
|---|---|
| Gum base | 20 |
| Powder sugar | 54 |
| Syrup (Water content 20%) | 18 |
| Sorbitol | 10 |
| Flavor | 2 |
| Emulsifier | 1 |

As a comparative example, the chewing gum was produced without adding derivative 2 in the composition 1. These were stored at room temperature for overnight (maturation).

### Sensory evaluation

3g of the chewing gum were chewed, and the change of the sweetness intensity felt in the mouse over the course of time was recorded. The sweetness intensity of the chewing gum of the comparative example disappeared after chewing for 4 minutes, and Egumi (a bitter taste in Japanese) and astringent taste were felt. However, the sweetness of the chewing gum which is mixed with the derivative 2 in the gum paste has been felt after chewing for 5 minutes, and continued effect of sweetness for 20 minutes was observed. And Egumi and astringent tastes derived from the gum paste were not felt.

### (Example 8) Production of the liquid sweetener composition by using the derivative 1

1.0 weight part of derivative 1 (the magnification of sweetness intensity is 22600 times) was added to 70 weight parts of water (the solubility of the derivative 1 in water at 25°C is 0.152g/100ml). After homogenizing by the homogenizer, 70 weight parts of isomerised sugar produced by Nippon Shokuhin Kakou Co. Ltd., the name of the product: "Fujikuraft" (water content 25%, the magnification of sweetness intensity: 0.75 times) were added, further homogenized and the suspension (slurry) comprising said derivative 1 was vacuum mixed by "Robokupe" (TK. SUPPLIES Co. Ltd.) with 2000 weight parts of "Fujikuraft" for 5 minutes, the sweetener composition in the form of suspension was produced. The solubilirty of the derivative 1 against "Fujikraft" at 25°C was 0.035g/100ml. And, the concentartion of the derivative 1 in the sweetener composition in the form of suspension is 0.047g/100ml (=1/2141; as the specific gravity is 1).

As 1 ml of the sweetener composition in the form of suspension has the sweetness intensity equivalent to 11.3* of sucrose, when 0.5 ml are dropped in the 140 ml of coffee which is a standard volume of a coffee cup, the coffee having the sweetness intensity equivalent to that of 4% sucrose is obtained.

### *:Calculation of the sweetness intensity

| | |
|---|---|
| from derivative 1 | 1 x 22600 |
| from "Fujikuraft" | (70+2000) x 0.75 |
| Total | 24152.5 |

Therefore, if it is 1g, specific gravity 1, the sweetness intensity is 11.3=24152.5/2141 per 1ml.

Although said sweetener composition in the form of suspension was poured into 20ml scale graduated cylinder, the top of the cylinder was wrapped by the transparent film for preventing the evaporation and it was stood still at room temperature for 60 days, the precipitation of derivative 1 can not be obtained. The viscosity of said sweetener composition in the form of suspension was 410 mPa.s (centipoise: 20°C, digital viscometer DVN-B type of Tokyo Keiki Co. Ltd.., rotor No.2, 30 rpm, 1 minute).

In the same way, when the suspension which was prepared by adding 1.0 weight part of derivative 1 to 70 weight parts of water and homogenizing the mixture by homogenizer, was stood still at room temperature for 7 days in the same way, all suspended substances were precipitated.

After producing said sweetener composition in the form of suspension, when the sensory evaluation was performed by comparing the 140 ml coffee which was added 0.5 ml of said sweetener composition with 140 ml coffee which was added 5.6g of sucrose (sucrose 4%) as a reference, any significant difference was not observed in the sweetness. And, said sweetener composition in the form of suspension was poured into the sealed container and stood still at room temperature for 60 days. After that, when the sensory evaluation was performed by comparing the 140 ml coffee which was added 0.5 ml of said sweetener composition with 140 ml coffee which was added 5.6g of sucrose as a reference in the same way, any significant difference was not observed in the sweetness, delicious coffee was obtained. From these results, it was found that the derivative 1 was not decomposed and it was stable.

When 50g of said suspension of the derivative (derivative content 0.0234g) were added to 500ml of water (25°C) with stirring (200rpm), and the time required to dissolve completely was measured, it took 50 seconds. When 0.0234g of the powder of'the derivative 1, as a reference product, were added to 500ml of water (25°C) with stirring (200rpm) in the same way, and the time required to dissolve completely was measured, it took not less than 3 minutes.

From the results of above Example 8, it was understood that the sweetener composition of the present invention was obtained stably, and it was also confirmed that the stability was increased extremely by adding the stabilizer such as isomerised sugar, compared with the case of water alone.

### (Example 9) Production of the liquid sweetener composition by using derivative 2

The following suspension medium was produced.

| Components | Weights ( g ) |
|---|---|
| D-sorbitol (70% aqueous solution) | 303 |
| Degassed distilled water | 45 |
| Sodium benzoate | 0.62 |
| Sodium carboxymethyl cellulose | 0.03 |
| Polysorbate 80 | 1.35 |
| Total | 350 |

In the components constituting the above composition, D-sorbitol which is used as a stabilizer for high solubility and high dispersibility, was expected both to improve the taste quality and to form the viscosity (a filler), and the degassed distilled water was used to remove the influence for the rate of precipitation of the derivative 2 by dissolved gases and dissolved ions. And, Sodium benzoate was used as a fungicide, Polysorbate 80 was used as a surfactant for improving the solubility of the derivative 2. Sodium carboxymethyl cellulose was used as a stabilizer for dispersion, particularly, a stabilizer for increasing the viscosity. The viscosity of this suspension was approximately 56 mPa.s (centipoise, measuring conditions: 20°C, rotor No.2, 30 rpm, 1 minute, digital viscometer DVN-B type of Tokyo Keiki Co. Ltd..).

58.3 g of the above suspension medium (dispersion medium) were fractionated into a 100ml-scale beaker, 0.160g of the powder of derivative 2 (the particle size [median diameter] was evened up to approximately 12 µm) were measured and added into the beaker for stirring by using a stirrer (at 20°C, for 30 minutes). After that, the suspension was transferred to the 50ml scale Meßzylinder from the beaker, the Meßzylinder was stood still, and the volume of the supernatant was measured at regular intervals of time (20°C). The solubility of the derivative 2 in water is 0.009g/100ml (25°C). As a reference, Aspartame was used in place of derivative 2

| Sample No. | Sweetener | Magnification of sweetness intensity (vs. sucrose) | Amount of use (g) | vs. dispersion medium 58.3g (%) |
|---|---|---|---|---|
| 1 | Derivative 2 | 5000 | 0.160 | 0.27 |
| 2 (Comparable example) | Aspartame | 200 | 4 | 6.86 |

The volumes of the supernatant (ml) at regular intervals of time are shown as follows.

| Sample No. | 0 hour | 24 hours | 48 hours | 72 hours | 3 months |
|---|---|---|---|---|---|
| 1 | 0 | 0 | 0 | 0 | 0 |
| 2 (Comparable example) | 0 | 9 | 18 | 25 | Precipitate all |

As a result, with respect to the product of the present invention, the precipitation of the supernatant was not observed. By using the derivative 2 in the form of suspension, the sweetener composition with a high intense sweetness in the form of suspension, which is more stable (without precipitation) and in which the sweetness is dispersed homogeneously, can be provided. As the stable sweetener composition in the form of suspension can be provided even under the low viscosity (for example, not more than 100mPa·s), the tabletop sweetener or the portable sweetener which is highly fluid and convenient when one or two drops of the sweetener is added in coffee and black tea. For example, when 0.5g of the obtained sweetener composition in the form of suspension are added to 140ml of coffee, the sweetness intensity equivalent to approximately 5% sucrose can be obtained. And, it is suitable for the use in the vending machine. Further, it is understood that it can be used for sprinkling on the sherbet as a sweetener, and imparting the sweetness after cocking.

When the sweetener composition with the same intensity of sweetness by using derivative 1 is wanted to produce, 0.035g of derivative 1 (4/22600) may be added to 58.3g of the above suspension medium (the dispersion medium) . In this case, derivative 1 is dissolved in the form of solution. When the sweetener composition is wanted to use in the form of solution considering the concentration for the respective object, it is not necessary to make any suspension. When 0.5g of the obtained said sweetener composition in the form of suspension are added to 140ml of coffee, the sweetness intensity equivalent to approximately 5% sucrose can be obtained. The solubility of derivative 1 in water is 0.152g/100ml (25°C).

Hereinafter, Production Examples of the aspartyl dipeptide ester derivatives which are used for the present invention are shown.

### (Production Example 1) Production of derivative 1 Synthesis of N-[N-[3-(3-hydroxy-4-methoxyphenyl) propyl]-L-α-aspartyl]-L-phenylalanine 1-methyl ester

To 485 mg (1.0 mmol) of N-t-butoxycarbonyl-β-o-benzyl-α-L-aspartyl-L-phenylalanine methyl ester, 5 ml of a 4N-HCl/dioxane solution were added and stirred at room temperature for one hour. The reaction solution was concentrated under reduced pressure. To the residue were added 30 ml of a 5%-aqueous solution of sodium hydrogen carbonate and extraction was made twice with 30 ml of ethyl acetate. An organic layer was washed with a saturated saline water and dried over anhydrous magnesium sulfate. Magnesium sulfate was filtered off and the liquid filtrate was concentrated under reduced pressure to yield 385 mg of β-o-benzyl-α-L-aspartyl-L-phenylalanine methyl ester, as a viscous oily substance.

385 mg (1.0 mmol) of the above β-o-benzyl-α-L-aspartyl -L-phenylalanine methyl ester were dissolved in 15 ml of tetrahydrofuran (THF) to yield a solution which was maintained at 0 °C. To this solution were added 268 mg (1.0 mmol) of 3-benzyloxy-4-methoxycinnamaldehyde, 0.060 ml (1.0 mmol) of acetic acid and 318 mg (1.5 mmol) of NaB(OAc)₃H and stirred for one hour at 0 °C and overnight at room temperature. To the reaction solution were added 50 ml of a saturated aqueous solution of sodium hydrogen carbonate and extraction was made twice with 30 ml of ethyl acetate. An organic layer was washed with a saturated saline water and dried over anhydrous magnesium sulfate. Magnesium sulfate was filtered off and the liquid filtrate was concentrated under reduced pressure. The residue was purified with preparative thin layer chromatography (PTLC) to yield 523 mg (0.82 mmol) of N-[N-[3-(3-benzyloxy-4-methoxyphenyl) propenyl]-β-o-benzyl-L-α-aspartyl]-L-phenylalanine 1-methyl ester as a viscous oily substance. To above 523 mg (0.82 mmol) of N-[N-[3-(3-benzyloxy-4-methoxyphenyl) propenyl]-β-o-benzyl-L-α-aspartyl]-L-phenylalanine 1-methyl ester were dissolved in a mixed solvent of 30 ml of methanol and 1 ml of water, and 200 mg of 10% palladium carbon (containing 50% of water) were added thereto. The resulting mixture was reduced at room temperature for three hours under a hydrogen atmosphere. The catalyst was filtered off and the filtrate was concentrated under reduced pressure. The residue was purified with PTLC to remove an odor adsorbed to yield 228 mg (0.48 mmol) of N-[N-[3-(3-hydroxy-4-methoxyphenyl) propyl]-L-α-aspartyl]-L-phenylalanine 1-methyl ester as a solid substance.
¹HNMR (DMSO-d₆) δ :1.50-1.60 (m, 2H), 2.15-2.40 (m,6H), 2.87-2.97 (dd, 1H), 3.05-3.13 (dd, 1H), 3.37-3.43 (m, 1H), 3.62 (s, 3H), 3.71 (s, 3H), 4.50-4.60 (m,1H), 6.52 (d, 1H), 6.60 (s,1H), 6.79 (d, 1H), 7.18-7.30 (m, 5H), 8.52 (d, 1H), 8.80 (brs, 1H).
ESI(Electrospray Ionization)-MS 459.2 (MH⁺).

### (Production example 2) Production of derivative 2

### Synthesis of N-[N-[3-(4-methoxyphenyl) propyl]-L-α-aspartyl]-L-phenylalanine 1-methyl ester

405 mg (2.5 mmol) of 4-methoxycinnamaldehyde, 735 mg (2.5 mmol) of aspartame and 350 mg of 10% palladium carbon (containing 50% of water) were added to a mixed solvent of 15 ml of methanol and 5 ml of water, stirred overnight at room temperature under a hydrogen atmosphere. The catalyst was filtered off and the filtrate was concentrated under reduced pressure. To the residue were added 30 ml of ethyl acetate, stirred for a while and then insoluble materials ware collected by filtration. After washing the collected insoluble materials with a little amount of ethyl acetate, 50 ml of a mixed solvent of ethyl acetate and methanol (5:2) were added to them and they were stirred for a while. Insoluble materials were removed by filtration, and the filtrate was concentrated until all the residue became the solid. This was dried under reduced pressure, and recrystalized in the mixed solvent of methanol and water, to obtain N-[N-[3-(4-methoxyphenyl) propyl]-L-α-aspartyl]-L-phenylalanine 1-methyl ester as a solid with a total yield of 43.4%.

### (Production Example 3) Production of derivative 3

### Synthesis of N-[N-[3-(3-hydroxy-4-methoxyphenyl)-3 -methylbutyl]-L-α-aspartyl]-L-phenylalanine 1-methyl ester

To 703 mg (1.45 mmol) of N-t-butoxycarbonyl-β-o -benzyl-(α-L-aspartyl-L-phenylalanine methyl ester, 10 ml of a 4N-HCl/dioxane solution were added and stirred at room temperature for one hour. The reaction solution was concentrated under reduced pressure. To the residue were added 50 ml of a 5%-aqueous solution of sodium hydrogen carbonate and extraction was made twice with 50 ml of ethyl acetate. An organic layer'was washed with a saturated saline wter and dried over anhydrous magnesium sulfate. Magnesium sulfate was filtered off and the liquid filtrate was concentrated under reduced pressure to yield 557 mg (1.45 mmo) of β-o-benzyl-α-L-aspartyl-L-phenylalanine methyl ester, as a viscous oily substance.

557 mg (1.45 mmol) of the above β-o-benzyl-α-L-aspartyl-L-phenylalanine methyl ester were dissolved in 15 ml of tetrahydrofuran (THF) to yield a solution which was maintained at 0 °C. To this solution were added 432 mg (1.45 mmol) of 3-(3-benzyloxy-4-methoxyphenyl)-3-methylbutyl aldehyde, 0.083 ml (1.45 mmol) of acetic acid and 462 mg (2.18 mmol) of NaB(OAc)₃H and stirred for one hour at 0 °C and overnight at room temperature. To the reaction solution were added 50 ml of a saturated aqueous solution of sodium hydrogen carbonate and extraction was made twice with 50 ml of ethyl acetate. An organic layer was washed with a saturated saline water and dried over anhydrous magnesium sulfate. Magnesium sulfate was filtered off and the liquid filtrate was concentrated under reduced pressure. The residue was purified with preparative thin layer chromatography (PTLC) to yield 832 mg (1.25 mmol) of N-[N-[3-(3-benayloxy-4-methoxyphenyl)-3-methylbutyl]-β-o-benzyl-L-a-aspartyl]-L-phenylalanine 1-methyl ester as a viscous oily substance. To above 832 mg (1.25 mmol) of N-[N-[3-(3-benzyloxy-4-methoxyphenyl)-3-methylbutyl]-β-o-benzyl-L-α-aspartyl]-L-phenylalanine 1-methyl ester were dissolved in a mixed solvent of 25 ml of methanol and 2 ml of water, and 350 mg of 10% palladium carbon (containing 50% of water) were added thereto. The resulting mixture was reduced at room temperature for three hours under a hydrogen atmosphere. The catalyst was filtered off and the filtrate was concentrated under reduced pressure. The residue was purified with PTLC to remove an odor adsorbed to yield 400 mg (0.82 mmol) of N-[N-[3-(3-hydroxy-4-methoxyphenyl)-3-methylbutyl]-L-α-aspartyl]-L-phenylalanine 1-methyl ester as a solid substance.
¹HNMR (DMSO-d₆) δ:1.14 (s,6H), 1.54-1.68(m,2H), 2.04-2.22 (m,3H), 2.24-2.34 (dd, 1H), 2.84-2.94 (dd, 1H), 3.00-3.08 (dd, 1H), 3.31-3.36 (m, 1H), 3.59 (s, 3H), 3.71 (s, 3H), 4.46-4.55 (m, 1H), 6.60-6.65 (dd, 1H), 6.73 (s, 1H), 6.80 (d, 1H), 7.10-7.28 (m,5H), 8.45 (d, 1H), 8.75 (brs, 1H).
ESI-MS 487.3 (MH⁺)

### (Production Example 4) Production of derivative 4 Synthesis of N-[N-[3-(3-methyl-4-hydroxyphenyl)-3-methylbutyl]-L-α-aspartyl]-L-phenylalanine 1-methyl ester

N-[N-[3-(3-methyl-4-hydroxyphenyl)-3-methylbutyl]-L-α-aspartyl]-L-phenylalanine 1-methyl ester was obtained as a solid substance, with a total yield of 63.2%, in the same way as in Production Example 3, except using 3- (3-methyl -4-benzyloxyphenyl)-3-methylbutyl aldehyde in place of 3-(3-benzyloxy-4-methoxyphenyl)-3-methylbutyl aldehyde.
¹HNMR (DMSO-d₆) δ:1.14 (s,6H), 1.59-1.68 (m,2H), 2.09 (s,3H), 2.09-2.18 (m,3H), 2.25 (dd,1H), 2.90 (dd,1H), 3.02 (dd,1H), 3.30-3.36 (m,1H), 3.59 (s,3H), 4.46-4.54 (m,1H), 6.68 (d,1H), 6.88 (dd,1H), 6.96 (s,1H), 6.14-6.73 (m,5H), 8.46 (d,1H), 9.01 (brs, 1H).
ESI-MS 471.4 (MH⁺)

### (Production Example 5) Production of derivative 5

### Synthesis of N-[N-[3-(4-methoxyphenyl)-3-methylbutyl]-L-α-aspartyl]-L-phenylalanine 1-methyl ester

N-[N-[3-(4-methoxyphenyl)-3-methylbutyl]-L-α-aspartyl]-L-phenylalanine 1-methyl ester was obtained as a solid substance, with a total yield of 72.2%, in the same way as in Production Example 3, except using 3-(4-methoxyphenyl)-3-methylbutyl aldehyde in place of 3-(3-benzyloxy-4-methoxyphenyl)-3-methylbutyl aldehyde.
¹HNMR (DMSO-d₆) δ:1.17 (s, 6H), 1.62-1.72 (m,2H), 2.04-2.20 (m, 3H), 2.24-2.34 (dd, 1H), 2.84-2.94 (dd, 1H), 2.95-3.07 (dd,1H), 3.30-3.35 (m, 1H), 3.51 (s, 3H), 3.70 (s,3H), 4.46-4.54 (m,1H), 6.83 (d,2H), 7.14-7.28 (m, 7H), 8.43 (d, 1H).
ESI-MS 471.3 (MH⁺)

### (Production Example 6) Production of derivative 6

### Synthesis of N-[N-[3-(4-hydroxyphenyl)-3-methylbutyl]-L-α-aspartyl]-L-phenylalanine 1-methyl ester

N-[N-[3-(4-hydroxyphenyl)-3-methylbutyl]-L-α-aspartyl]-L-phenylalanine 1-methyl ester was obtained as a solid substance, with a total yield of 64.5%, in the same way as in Production Example 3, except using 3-(4-benzyloxyphenyl)-3-methylbutyl aldehyde in place of 3-(3-benzyloxy-4-methoxyphenyl)-3-methylbutyl aldehyde.
¹HNMR (DMSO-d₆) δ:1.15 (s, 6H), 1.58-1.72 (m,2H), 2.04-2.20 (m, 3H), 2.24-2.34 (dd, 1H), 2.85-2.94 (dd, 1H), 3.00-3.08 (dd,1H), 3.30-3.36 (m, 1H), 3.59 (s, 3H), 4.46-4.55 (m,1H), 6.67 (d,2H), 7.07 (d, 2H), 7.10-7.27 (m, 5H), 8.44 (d, 1H), 9.15 (brs,1H).
ESI-MS 457.3 (MH⁺)

### (Production Example 7) Production of derivative 7

### Synthesis of N-[N-[3-(2-hydroxy-4-methoxyphenyl) propyl]-L-α-aspartyl]-L-phenylalanine 1-methyl ester

N-[N-[3-(2-hydroxy-4-methoxyphenyl) propyl]-L-α-aspartyl]-L-phenylalanine 1-methyl ester was obtained as a solid substance, with a total yield of 54.4%, in the same way as in Production Example 1, except using 2-benzyloxy-4-methoxycinnamaldehyde in place of 3-benzyloxy-4-methoxycinnamaldehyde.
¹HNMR (DMSO-d₆) δ : 1.52-1.57 (m,2H), 2.20-2.31 (m, 2H), 2.26-2.41 (m, 4H), 2.88-3.11 (m, 2H), 3.41-3.43 (m, 1H), 3.62 (s, 3H), 3.65 (s, 3H), 4.53-4.59 (m,1H), 6.28-6.36 (m, 2H), 6.88-6.90 (d,1H), 7.19-7.29 (m, 5H), 8.55 (d, 1H).
ESI-MS 459.3 (MH⁺)

### (Production Example 8) Production of derivative 8

### Synthesis of N-[N-[3-(3-methyl-4-hydroxyphenyl) propyl]-L-α-aspartyl]-L-phenylalanine 1-methyl ester

N-[N-[3-(3-methyl-4-hydroxyphenyl) propyl]-L-α-aspartyl]-L-phenylalanine 1-methyl ester was obtained as a solid substance, with a total yield of 32.2%, in the same way as in Production Example 1, except using 3-methyl-4-benzyloxycinnamaldehyde in place of 3-benzyloxy-4-methoxycinnamaldehyde.
¹HNMR (DMSO-d₆) δ : 1.50-1.58 (m,2H), 2.08 (s, 3H), 2.09-2.30 (m, 2H), 2.26-2.38 (m, 4H), 2.89-3.09 (m, 2H), 3.35-3.42 (m, 1H), 3.62 (s, 3H), 4.54-4.59 (m,1H), 6.65-6.83 (m, 3H), 7.19-7.28 (m, 5H), 8.52 (d, 1H), 9.04 (brs, 1H).
ESI-MS 443.4 (MH⁺)

### (Production Example 9) Production of derivative 9

### Synthesis of N-[N-[3-(2, 4-dihydroxyphenyl) propyl]-L-α-aspartyl]-L-phenylalanine 1-methyl ester

N-[N-[3-(2, 4-dihydroxyphenyl) propyl]-L-α-aspartyl]-L-phenylalanine 1-methyl ester was obtained as a solid substance, with a total yield of 42.6%, in the same way as in Production Example 1, except using 345 mg (1.0 mmol) of 3-(2,4-dibenzyloxyphenyl)-2-propenylaldehyde in place of 268 mg (1.0 mmol) of 3-benzyloxy-4-methoxycinnamaldehyde.
ESI-MS 445.3 (MH⁺)

### Effect of the Invention

According to the present invention, a solid sweetener composition (including the form of solid food) which is produced by mixing homogeneously an aspartyl dipeptide ester derivative (one kind or more; which may be in the salt form) contained in the above general formula (2), particularly (1), with a filler (including a bulking agent and a carrier and so on) in the form of solution (if necessary, including the drying process), wherein the sweetness of the derivative is dispersed and mixed homogeneously, can be provided.

By the present invention, by mixing homogeneously with said various fillers used for the solid sweetener composition of the present invention, a solid sweetener composition with a high intense sweetness having a homogeneous taste characteristics which can not be obtained by the single use of one kind or more of the derivatives represented by the above general formula (2), particularly (1), can be provided. It can be used as a solid sweetener and solid food (powdered juice and so on), and as a sweetness imparting agent for the product such as food and drink which is required the homogeneous sweetness.

Furthermore, according to the present invention, the stable solution or the suspension (a liquid sweetener composition of the present invention) as a sweetener composition, which comprises one kind or more of the aspartyl dipeptide ester derivative (which may be in the salt form) represented by the above general formula (2), particularly (1), in the edible medium such as water, can be obtained.

This liquid sweetener composition is superior in solubility, or particularly in dispersibility, and can be stored in a small place for a long time, having a.superior in operation, without problems of scattering and so on. It can be used simply as a sweetener and food and drink, or as a sweetness imparting agent for food and drink and so on. For example, although it exerts the superiority as a sherbet, a syrup and a sweetener for the vending machine, it can be applicable widely without any limitation of these products, for various products in need of imparting sweetness.

## Claims

1. A solid sweetener composition comprising an aspartyl dipeptide ester derivative, which may be in the salt form, represented by the following general formula (1), and a solid filler,
wherein said composition is produced in at least a step of mixing said derivative in the form of a solution during manufacture, thereby said derivative being mixed and dispersed homogeneously: 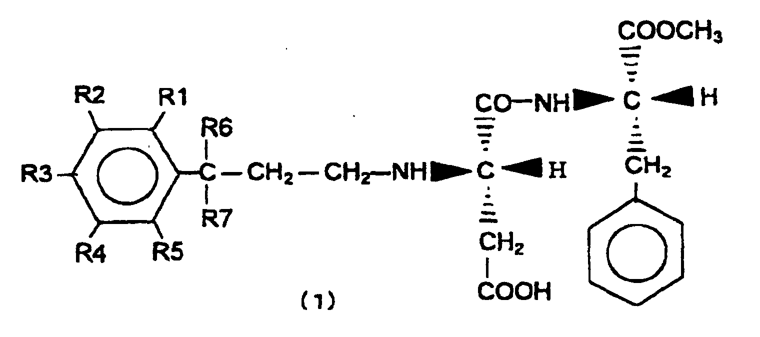 wherein R₁, R₂, R₃, R₄ and R₅ are independent from each other, and each denotes any one selected from the group consisting of a hydrogen atom, a hydroxyl group, a methoxy group and a methyl group, and R₆ and R₇ are independent from each other, and each denotes a hydrogen atom or a methyl group, respectively,
and when R₆ and R₇ denote different substituents each other, the carbon atom to which these substituents are linked may be in the (R), (S) or (RS) configuration.

2. The sweetener composition as defined in claim 1, wherein the sweetness intensity of said aspartyl dipeptide ester derivative is more than 4,000 times that of sucrose.

3. The sweetener composition as defined in claim 2, wherein in said general formula, R₃ is a hydroxyl group or a methoxy group, and R₄ and R₅ are a hydrogen atom.

4. The sweetener composition as defined in claim 3, wherein R₁ in said general formula is a hydroxyl group.

5. The sweetener composition as defined in claim 3, wherein R₁ in said general formula is a hydrogen atom.

6. The sweetener composition as defined in claim 4, wherein R₂, R₆ and R₇ in said general formula are a hydrogen atom.

7. The sweetener composition as defined in claim 5, wherein R₂ in said general formula is a hydrogen atom, a hydroxyl group or a methyl group.

8. The sweetener composition as defined in claim 1, wherein said aspartyl dipeptide ester derivative is at least one of the derivatives 1 to 9 wherein R₁ to R₇ in said general formula denote the following substituents:
| Derivative No. | R₁ | R₂ | R₃ | R₄ | R₅ | R₆ | R₇ |
|---|---|---|---|---|---|---|---|
| 1 | H | OH | OCH₃ | H | H | H | H |
| 2 | H | H | OCH₃ | H | H | H | H |
| 3 | H | OH | OCH₃ | H | H | CH₃ | CH₃ |
| 4 | H | CH₃ | OH | H | H | CH₃ | CH₃ |
| 5 | H | H | OCH₃ | H | H | CH₃ | CH₃ |
| 6 | H | H | OH | H | H | CH₃ | CH₃ |
| 7 | OH | H | OCH₃ | H | H | H | H |
| 8 | H | CH₃ | OH | H | H | H | H |
| 9 | OH | H | OH | H | H | H | H |

9. The sweetener composition as defined in claim 1, wherein said solid filler is at least one of the compounds contained in the group consisting of sugar, sugar alcohol, oligosaccharide and polysaccharide.

10. The sweetener composition as defined in claim 9, wherein said sugar comprises sucrose (including derivative of sucrose), invert sugar, isomerized sugar, glucose, fructose, lactose, malt sugar, D-xylose and isomerized lactose; said sugar alcohol comprises maltitol, sorbitol, mannitol, erythritol, xylitol, lactitol, palatinit, and reduced starch sugar; said oligosaccharide comprises fructooligosaccharide, maltooligosaccharide, isomaltooligosaccharide, galactooligosaccharide, soy been oligosaccharide and lactooligosaccharide; and said polysaccharide comprises glucomannan, dietary fiber (including enzyme decomposition product(s) of guar gum, non-digestible dextrin, polydextrin and so on) and starch (including dextrin, soluble starch, modified starch and so on).

11. The sweetener composition as defined in claim 1, wherein a ratio of said aspartyl dipeptide ester derivative to a total amount of said aspartyl dipeptide ester derivative and said filler is in the range of 2 ppm to 95% by weight.

12. The sweetener composition as defined in claim 1, obtained by drying the solution of said composition dissolved homogeneously.

13. The sweetener composition as defined in claim 1, solidified by any one of the methods consisting of condensation drying, spray drying, freeze drying, extrusion granulation and absorption to forming sugar.

14. The sweetener composition as defined in claim 1, wherein a solvent constituting said solution is water, alcohol, or a homogeneously mixed solvent comprising water and/or alcohol.

15. The sweetener composition as defined in claim 1, obtained by coating said filler homogeneously with a solution comprising said aspartyl dipeptide ester derivative.

16. The sweetener composition as defined in claim 1, which is a sweetened solid food or so on.

17. The sweetener composition as defined in claim 1, which is at least one selected from the group consisting of a sweetener, juice, coffee, cocoa, powdered cola, black tea, health care food, chewing gum, chocolate, medicine and solid tooth paste.

18. A solid or liquid food and drink or so on, obtained by using the sweetener composition as defined in any one of claims 1 to 17.

19. A liquid sweetener composition comprising a solution dissolving at least one of aspartyl dipeptide ester derivatives, which may be in the salt form, represented by the following general formula (1) in an edible medium: 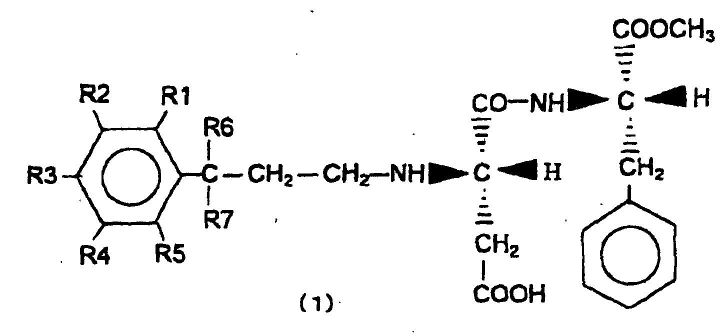 wherein R₁, R₂, R₃, R₄ and R₅ are independent from each other, and each denotes any one selected from the group consisting of a hydrogen atom, a hydroxyl group, a methoxy group and a methyl group, and R₆ and R₇ are independent from each other, and each denotes a hydrogen atom or a methyl group, respectively,
and when R₆ and R₇ denote different substituents each other, the carbon atom to which these substituents are linked may be in the (R), (S) or (RS) configuration.

20. The sweetener composition as defined in claim 19, wherein the sweetness intensity of said aspartyl dipeptide ester derivative is more than 4,000 times that of sucrose.

21. The sweetener composition as defined in claim 20, wherein in said general formula, R₃ is a hydroxyl group or a methoxy group, and R₄ and R₅ are a hydrogen atom.

22. The sweetener composition as defined in claim 21, wherein R₁ in said general formula is a hydroxyl group.

23. The sweetener composition as defined in claim 21, wherein R₁ in said general formula is a hydrogen atom.

24. The sweetener composition as defined in claim 22, wherein R₂, R₆ and R₇ in said general formula are a hydrogen atom.

25. The sweetener composition as defined in claim 23, wherein R₂ in said general formula is a hydrogen atom, a hydroxyl group or a methyl group.

26. The sweetener composition as defined in claim 19, wherein said aspartyl dipeptide ester derivative is at least one of the derivatives 1 to 9 wherein R₁ to R₇ in said general formula denote the following substituents:
| Derivative No. | R₁ | R₂ | R₃ | R₄ | R₅ | R₆ | R₇ |
|---|---|---|---|---|---|---|---|
| 1 | H | OH | OCH₃ | H | H | H | H |
| 2 | H | H | OCH₃ | H | H | H | H |
| 3 | H | OH | OCH₃ | H | H | CH₃ | CH₃ |
| 4 | H | CH₃ | OH | H | H | CH₃ | CH₃ |
| 5 | H | H | OCH₃ | H | H | CH₃ | CH₃ |
| 6 | H | H | OH | H | H | CH₃ | CH₃ |
| 7 | OH | H | OCH₃ | H | H | H | H |
| 8 | H | CH₃ | OH | H | H | H | H |
| 9 | OH | H | OH | H | H | H | H |

27. The sweetener composition as defined in claim 19 comprising at least one of the compounds contained in the group consisting of sugar, sugar alcohol, and oligosaccharide.

28. The sweetener composition as defined in claim 27, wherein said sugar comprises sucrose (including derivative(s) of sucrose), invert sugar, isomerized sugar, glucose, fructose, lactose, malt sugar, D-xylose and isomerized lactose, said sugar alcohol comprises maltitol, sorbitol, mannitol, erythritol, xylitol, lactitol, palatinit, and hydrogenated starch hydrolysate; and said oligosaccharide comprises fructooligosaccharide, maltooligosaccharide, isomaltooligosaccharide, galactooligosaccharide, soy been oligosaccharide and lactooligosaccharide.

29. The sweetener composition as defined in claim 19, wherein said edible medium is a liquid medium, and which is in the form of a suspension comprising at least one of said derivatives in a concentration higher than the solubility thereof in said liquid medium.

30. The sweetener composition as defined in claim 29, obtained by mixing a suspension comprising at least one of said derivatives in a concentration higher than the solubility thereof in a liquid medium, and a liquid medium.

31. The sweetener composition as defined in claim 29, wherein said liquid medium is water or an aqueous solution comprising water and at least one of the compounds contained in the group consisting of sugar, sugar alcohol, and oligosaccharide.

32. The sweetener composition as defined in claim 30, wherein a process for said mixing is conducted under reduced pressure.

33. The sweetener composition as defined in claim 31, obtained by mixing at least one of said derivatives and a liquid medium, and then, mixing therewith at least one of the compounds contained in the group consisting of sugar, sugar alcohol, and oligosaccharide, with or without water.

34. The sweetener composition as defined in any one of claims 29 to 33, which is in the form of homogeneous suspension.

35. The sweetener composition as defined in any one of claims 19 to 34, which is in the form of a sweetener, or food and drink.

36. Food and drink, or an another sweetened product obtained by using the sweetener composition as defined in any one of claims 19 to 35.
